# EUROPEAN PATENT APPLICATION

(11) **EP 4 817 540 A1**
(43) Date of publication of application: **07.10.2026**
(21) Application number: 26170018.1
(22) Date of filing: 31.03.2026
(51) Int. Cl.: A61B 17/84, A61F 2/30, A61F 2/44

(54) **BONE ANCHOR FOR SECURING AN INTERVERTEBRAL IMPLANT AND CORRESPONDING SPINAL STABILIZATION SYSTEM**

(30) Priority: 31.03.2025 US 202519095512
(71) Applicant: Globus Medical, Inc., Audubon, PA 19403 (US)
(72) Inventor: BAKEY, Matthew, HARLEYSVILLE, 19438 (US); ZAPPACOSTA, Jason, PHILADELPHIA, 19130 (US)
(74) Representative: Morabito, Sara

(57) **Abstract**

Bone anchors, expandable intervertebral fusion devices, systems, instruments, and methods thereof. The anchor may include a spherical head and a curved blade extending therefrom. The spherical head may define a non-threaded opening including a vertical slot in communication with an internal spherical pocket, which interfaces with a corresponding non-threaded tip of a single anchor impactor instrument. The anchor may attach to the instrument via a quarter-turn connection, which provides navigation for straight and angled insertion and/or removal of the anchors.

## Description

### FIELD

The present disclosure generally relates to devices and methods for promoting an intervertebral fusion, and more particularly relates to anchors for securing expandable fusion devices and related systems, instruments, and methods.

### BACKGROUND

A common procedure for handling pain associated with intervertebral discs that have become degenerated due to various factors, such as trauma or aging, is the use of intervertebral fusion devices for fusing one or more adjacent vertebral bodies. Generally, to fuse the adjacent vertebral bodies, the intervertebral disc is first partially or fully removed. An intervertebral fusion device is then inserted between neighboring vertebrae to maintain normal disc spacing and restore spinal stability, thereby facilitating an intervertebral fusion. In some cases, the intervertebral fusion device may be secured with one or more bone anchors, such as bone screws.

There are drawbacks, however, with existing anchors including inadequate fixation, potential expulsion, difficult installation along a range of height and lordosis options, and/or complicated instruments for installation. For example, traditional anchor guides hold the anchors at specific heights and trajectories, which are incompatible with adjustable lordosis spacers as the interbody can be expanded along a range of height and lordosis options. As such, there exists a need for improved anchors capable of providing fixation and expulsion resistance with improved insertion and removal instruments and techniques.

### SUMMARY

To meet this and other needs, implants, systems, instruments, and methods for performing intervertebral fusion and spine stabilization are provided. In particular, bone anchors may be compatible with intervertebral implants, such as adjustable lordosis interbody spacers installed from an ALIF (anterior lumbar interbody fusion) approach. The expandable implants are configured to be inserted into the intervertebral disc space at a minimized height, and then expanded axially to restore height loss in the disc space, obtain normal spinal alignment, and/or distribute the load across the vertebral endplates. The implant may also change in lordotic angulation independently from its expansion. One or more bone anchors may be inserted through the endplates of the expandable implant to ensure the implant remains securely fixed within the vertebral space. As such, the bone anchors are configured to provide immediate stabilization, fixation, and resist expulsion forces that might dislodge the implant. In contrast to prior designs where bone anchors utilized a threaded bore for threaded engagement to an instrument, the bone anchors define a non-threaded slot configured to engage seamlessly with a corresponding inserter tip. The inserter tip may be secured to the non-threaded slot, for example, with a quarter-turn connection to the anchor, providing for freehand installation of each anchor. The non-threaded attachment interface significantly enhances the ease of attachment, insertion, and/or removal of the anchors, thereby streamlining the surgical process.

According to one embodiment, a bone anchor for securing an intervertebral implant includes a spherical head and a curved blade extending therefrom. The spherical head defines a non-threaded opening including a vertical slot in communication with an internal spherical pocket defined within the spherical head. The vertical slot has a narrow width defined between opposing flat sides with an elongated length longer than the width. The vertical slot is aligned with a longitudinal axis of the anchor. The spherical head includes a raised bump located beneath the vertical slot creating a thickened wall area. The raised bump has a radius smaller than the radius of the spherical head and a width greater than the width of the vertical slot.

The anchor may include one or more of the following features. The vertical slot may have an obround shape. The vertical slot may be offset toward a top of the spherical head such that a top end of the vertical slot cuts deeper into the spherical head relative to a bottom end of the vertical slot. The internal spherical pocket has a diameter greater than the width of the vertical slot, thereby forming an overhang beneath the sides of the vertical slot. Each side of the vertical slot may defines symmetric lips, and an apex of each lip may be truncated with a flat. The curved blade may include an elongate fin that ends away from a concave surface of the blade such that the elongate fin is generally orthogonal to the blade.

According to one embodiment, a spine stabilization system includes an expandable implant and bone anchor. The expandable implant has an upper endplate, a lower endplate, and an actuator assembly including a moveable anterior actuator, a moveable posterior actuator, and a stationary posterior actuator positioned between the upper and lower endplates to independently control anterior and posterior heights of the implant. The bone anchor is insertable through the upper or lower endplate and into adjacent bone. The bone anchor has a spherical head and a curved blade extending therefrom. The spherical head defines a non-threaded opening including a vertical slot in communication with an internal spherical pocket defined within the spherical head. The vertical slot has a narrow width defined between opposing flat sides with an elongated length longer than the width. The vertical slot is aligned with a longitudinal axis of the anchor. The spherical head includes a raised bump located beneath the vertical slot creating a thickened wall area. The raised bump has a radius smaller than the radius of the spherical head and a width greater than the width of the slot.

The spine stabilization system may include one or more of the following features. The upper and/or lower endplates may define a socket configured to receive the spherical head of the bone anchor, which guides the anchor into the adjacent bone. The socket may have a partial spherical cut, which provides a congruent surface for the spherical head of the anchor to permit rotation of the anchor within the socket. The socket may define a stop, which engages with the raised bump to limit rotation of the anchor, for example, for up to 20 degrees of rotation for the anchor within the socket. The expandable implant may include a blocking screw, which when rotated, covers a portion of the spherical head of the anchor, thereby preventing the anchor from backing out after insertion, and the raised bump prevents the non-threaded opening of the anchor from aligning with the blocking screw.

According to another embodiment, a method of implanting an orthopedic implant includes one or more of the following steps in any suitable order: (a) inserting an orthopedic implant between adjacent vertebrae, the orthopedic implant comprising a first endplate defining a first socket, a second endplate defining a second socket, and an actuator assembly including a moveable anterior actuator, a moveable posterior actuator, and a stationary posterior actuator positioned between the upper and lower endplates to independently control anterior and posterior heights of the implant; (b) expanding the implant to adjust height and/or lordosis; (c) attaching a first anchor to a single anchor impactor instrument having a non-threaded distal tip configured to mate with a non-threaded opening in the anchor, the instrument includes an inner shaft positioned through an outer sleeve, the inner shaft includes an elongated tip having curved sides with at least one flat side configured to mate with a corresponding flat side within a vertical slot of the anchor, the outer sleeve includes a distal end with a protruding rim, wherein the first anchor is attached to the instrument by rotating the inner shaft, which rotates the anchor such that the protruding rim of the outer sleeve fits under an overhang along sides of the vertical slot, thereby securing the first anchor to the instrument; (d) pivoting the first anchor along an axis of the vertical slot to accommodate a straight or angled trajectory for the first anchor; and (e) inserting the first anchor into the first socket of the first endplate to engage an upper vertebra. The inner shaft may include a ball detent configured to engage with divots within a housing of the outer sleeve such that the inner shaft is held in one of two relative positions defined by the divots when the inner shaft is rotated with respect to the outer sleeve. The inner shaft may rotate 90 degrees relative to the stationary outer sleeve, which rotates the first anchor 90 degrees to secure the first anchor to the instrument. The vertical slot may have a length, which permits the anchor to pivot based on limits imposed by ends of the vertical slot. The method may further include (f) attaching a second anchor to the single anchor impactor instrument by rotating the inner shaft and the anchor such that the protruding rim of the outer sleeve fits under the overhang along the sides of the vertical slot, thereby securing the second anchor to the instrument; (g) inserting the second anchor into the second socket of the second endplate to engage a lower vertebra; and/or (h) disassembling the inner shaft from the outer sleeve by depressing a release button within the outer sleeve to disengage from the central shaft.

According to another embodiment, a single anchor impactor instrument system includes an anchor and an instrument. The anchor has a spherical head and a curved blade extending therefrom. The spherical head defines a non-threaded opening including a vertical slot in communication with an internal spherical pocket defined within the spherical head. The vertical slot has flat sides forming an overhang with respect to the internal spherical pocket. The instrument has a non-threaded distal tip configured to mate with the non-threaded opening in the anchor. The instrument includes an inner shaft positionable through an outer sleeve. The inner shaft includes an elongated tip with a truncated distal-most end. The elongated tip has curved sides with at least one flat side configured to mate with one of the flat sides of the vertical slot when inserted therein. The outer sleeve includes a distal end with a protruding rim. When the inner shaft rotates the anchor, the protruding rim of the outer sleeve fits under the overhang along the sides of the vertical slot, thereby securing the anchor to the instrument.

The single anchor impactor instrument system may include one or more of the following features. The inner shaft may be configured to rotate 90 degrees relative to the outer sleeve, thereby also rotating the anchor 90 degrees about a long axis of the instrument while the outer sleeve remains stationary to secure the anchor to the instrument. The protruding rim may have a curved surface, and when the anchor is rotated, the curved surface of the rim of the outer sleeve fits within the internal spherical pocket and under the overhang along the sides of the vertical slot. The protruding rim may include a first rim portion and a second rim portion on opposite sides of the elongated tip of the inner shaft. The vertical slot may have a length, which is longer than a length of the elongated tip, which permits the anchor to pivot on the non-threaded distal tip. The anchor may be permitted to pivot up to 22 degrees on the non-threaded distal tip to facilitate angled insertion of the anchor. The truncated distal-most end of the elongated tip may have a curvature congruent with the curvature of the internal spherical pocket such that when the inner shaft is fully inserted into the non-threaded opening, the truncated distal-most end of the elongated tip bottoms out within the internal spherical pocket.

According to another embodiment, a single anchor impactor instrument system includes an anchor and an instrument. The anchor has a spherical head and a curved blade extending therefrom. The spherical head defines a non-threaded opening including a vertical slot in communication with an internal spherical pocket defined within the spherical head. The instrument has a non-threaded distal tip configured to mate with the non-threaded opening in the anchor. The instrument includes an inner shaft configured to rotate relative to a stationary outer sleeve. The inner shaft has a rear housing and a central shaft extending therethrough. The central shaft retains a shuttle and multiple washers. The outer sleeve has a housing and a tubular body for receiving the inner shaft therethrough. A lever is pivotably connected to the central shaft and the rear housing of the inner shaft. The shuttle and washers are configured to slide axially over the central shaft when the lever is actuated and the central shaft is moved axially. The instrument is configured to function in three positions: (a) in a first position, the instrument is ready for attachment to the anchor by extending the lever outward such that the non-threaded distal tip is oriented to fit into the vertical slot of the non-threaded opening; (b) in a second position, the anchor is attached to the instrument in an unlocked position by rotating the inner shaft and lever 90 degrees relative to the first position to secure the non-threaded distal tip to the non-threaded opening in the anchor while permitting angulation of the anchor along an axis of the vertical slot; and (c) in a third position, the anchor is locked to the instrument by pivoting the lever flush against the outer sleeve, which pulls back the central shaft, pushes the shuttle forward, compresses the washers, and keeps tension on the central shaft to hold the anchor tight against the non-threaded distal tip of the instrument.

The single anchor impactor instrument system may include one or more of the following features. The first and second positions may be defined by a ball detent configured to engage with divots within the housing of the outer sleeve such that the inner shaft is held in one of the two relative positions defined by the divots. A spring may be positioned around the central shaft and is captured by a cap, which fits inside the rear housing, and the spring biases the central shaft forward. The washers may include spring washers, which are compressed to lock the anchor to the instrument. The lever may be pivotably connected to the central shaft and the rear housing of the inner shaft by a pin, which acts as a pivot axis for the lever. In the first and second positions, the lever may be generally orthogonal to a long axis of the instrument, and in the third position, the lever may be generally parallel to the long axis of the instrument.

According to another embodiment, a single anchor impactor instrument includes an inner shaft and outer sleeve aligned along an instrument axis. The inner shaft has a rear housing and a central shaft extending therethrough. The central shaft retains a shuttle and multiple washers. The outer sleeve has a housing and a tubular body for receiving the inner shaft therethrough. The instrument includes a lever for locking an anchor to the instrument. The lever is pivotably connected to the central shaft and the rear housing of the inner shaft by a lever pin. The shuttle and washers are configured to slide axially over the central shaft when the lever is actuated and the central shaft is moved axially. The instrument includes a ball detent within the rear housing of the inner shaft configured to engage with divots within the housing of the outer sleeve such that the inner shaft is held in one of two relative positions when rotated with respect to the outer sleeve.

The single anchor impactor instrument may include one or more of the following features. The inner shaft may be configured to rotate 90 degrees while the outer sleeve remains stationary. The instrument may include a spring retained by a cap, which fits inside the rear housing of the inner shaft, to bias the central shaft forward. The instrument may include a keying tab located on an underside of the rear housing, which fits into a corresponding keying recess in the housing of the outer sleeve to ensure the lever is properly aligned. The instrument may include a release button with an opening for receiving the central shaft therethrough configured for disassembling the inner shaft from the outer sleeve. The central shaft may include a shoulder beneath the rear housing to define an engagement area for the release button. The washers may be located between the release button and the shuttle, and the shuttle may be positioned between the washers and the lever pin.

According to yet another embodiment, a kit may include a plurality of anchors of different sizes, expandable implants of different sizes and configurations, k-wires, and other components for performing the procedure. The kit may further include one or more instruments suitable for installing and/or removing the implants, such as insertion devices or drivers, expansion instruments, removal devices, anchor installation tools, and other tools and devices which may be suitable for surgery.

### BRIEF DESCRIPTION OF THE DRAWINGS

The present embodiments will become more fully understood from the detailed description and the accompanying drawings, wherein:
FIGS. 1A-1B illustrate perspective views of bone anchors having a spherical head and curved blade of varying lengths according to one embodiment;
FIGS. 2A-2C illustrate front, top, and cross-sectional views, respectively, of the bone anchor having an internal spherical pocket accessible via a vertical slot according to one embodiment;
FIGS. 3A-3B illustrate front and perspective views, respectively, of an expandable implant in an expanded position with bone anchors for securing the implant to adjacent vertebrae according to one embodiment;
FIGS. 4A-4B illustrate a close-up view of the spherical head of the anchor including an anti-rotation bump and a front view of the anchor positioned through the upper endplate of the expandable implant where the anti-rotation bump limits rotation of the anchor according to one embodiment;
FIGS. 5A-5C illustrate perspective views of an adjustable lordosis expandable implant in a fully collapsed position, at maximum expansion, and at maximum lordosis, respectively, according to one embodiment;
FIGS. 6A-6C illustrate side views of the adjustable lordosis expandable implant in the fully collapsed position, at maximum expansion, and at maximum lordosis, respectively, according to one embodiment;
FIG. 7 shows a close-up view of a non-threaded tip of an anchor insertion instrument for installing the bone anchor according to one embodiment;
FIGS. 8A-8B show a cross-sectional view of the insertion instrument positioned in the internal spherical pocket within the spherical head of the bone anchor, which is rotated to attach or detach from the anchor, while providing for straight or angled insertion and/or removal of the anchor according to one embodiment;
FIG. 9 shows an exploded view of a single anchor insertion and/or removal instrument according to one embodiment;
FIG. 10 shows a close-up cross-sectional view of the single anchor instrument according to one embodiment;
FIGS. 11A-11B show a perspective view of the anchor instrument and a close-up view of the distal tip when the instrument is ready to attach to the bone anchor;
FIGS. 12A-12B show a perspective view of the anchor instrument and a close-up view of the distal tip when the anchor is attached, but not locked to the instrument;
FIGS. 13A-13B show a perspective view of the anchor instrument and a close-up view of the distal tip when the anchor is attached and locked to the instrument; and
FIGS. 14A-14B show the inner shaft and rear housing assembly and outer sleeve of the anchor impactor, respectively, which may be disassembled for cleaning according to one embodiment.

### DETAILED DESCRIPTION

Embodiments of the disclosure are generally directed to devices, systems, instruments, and methods for intervertebral fusion and spine stabilization. Specifically, bone anchors may be installed into intervertebral implants to form standalone implants without the need for supplemental fixation (e.g., additional screws, rods, etc.). The implants may include expandable implants, such as adjustable lordosis interbody spacers installed from an ALIF (anterior lumbar interbody fusion) approach. The bone anchors may be positioned through the implant and into adjacent bone to ensure the implant remains securely fixed within the vertebral space. The bone anchors may include a spherical head with a curvilinear blade, which helps to prevent expulsion due to biomechanical forces exerted by the spine. Unlike prior anchors with a threaded bore necessitating threaded engagement with an instrument that can be difficult to attach in-situ, each bone anchor may define a non-threaded slot, which engages with a corresponding inserter tip for improved attachment, insertion, and removal functionality.

The expandable implants may be configured to be inserted into the intervertebral disc space at a minimized height, and then expanded axially to restore height loss, provide normal spinal alignment, and/or distribute the load across the vertebral endplates. The implant may also change in lordotic angulation independently from its height expansion. After the implant is positioned between the vertebrae and expanded to the desired height and/or lordosis, the bone anchors are inserted through respective guide channels or holes in the implant. The channels or holes may help to guide the respective anchors into the adjacent bone, for example, to optimize contact with the strongest areas of bone, enhancing the overall stability of the implant. By anchoring the implant firmly to the vertebral bone, the expulsion forces are effectively countered, thereby maintaining the integrity of the spinal alignment and facilitation fusion. A spinal fusion is typically employed to eliminate pain caused by motion of degenerated disc material. Upon successful fusion, a fusion device becomes permanently fixed within the intervertebral disc space.

Minimally invasive surgery (MIS) may be used to preserve muscular anatomy by only causing disruption where necessary. The benefit of the MIS surgical approach is that it can reduce post-operative pain and improve recovery time for patients. In one embodiment, the expandable fusion device and/or anchors can be configured to be placed down an endoscopic tube and into the surgical target site. By way of example, the surgical site may be an intervertebral disc space situated between two adjacent vertebrae. Although particularly suited for use in an anterior lumbar interbody fusion (ALIF), it will be readily appreciated by those skilled in the art that the implant may be employed in any number of suitable orthopedic approaches and procedures, such as direct lateral where coronal deformity is encountered. Other approaches may include but are not limited to posterior, lateral, anterolateral, posterolateral, or transforaminal approaches to the lumbar spine, cervical spine, or thoracic spine, as well as any non-spine application, such as treatment of bone fractures and the like. The terms implant, interbody, interbody implant, fusion device, spacer, and expandable device may be used interchangeably herein.

Components of all of the devices disclosed herein may be manufactured of any suitable materials including metals (e.g., titanium), metal alloys (e.g., stainless steel, cobalt-chromium, and titanium alloys), ceramics, plastics, plastic composites, or polymeric materials (e.g., polyether ether ketone (PEEK), polyphenylene sulfone (PPSU), polysulfone (PSU), polycarbonate (PC), polyetherimide (PEI), polypropylene (PP), polyacetals, or mixtures or co-polymers thereof), and/or combinations thereof. In some embodiments, the devices may include radiolucent and/or radiopaque materials. The components can also be machined and/or manufactured using any suitable techniques (e.g., additive manufacturing or 3D printing).

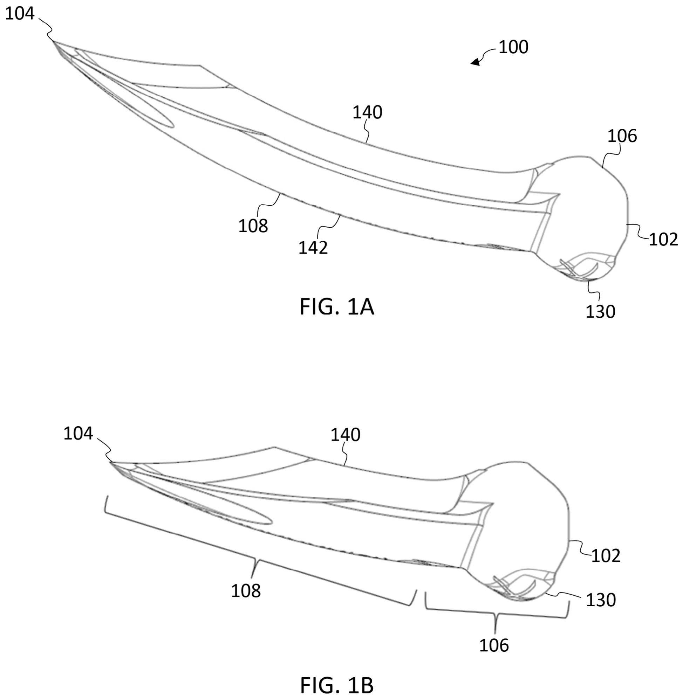

Turning now to the drawing, where like reference numerals may refer to like elements, FIGS. 1A-1B illustrate bone anchor 100 of varying lengths according to one embodiment. The bone anchors 100 may form part of a standalone intervertebral implant system, for example, entering the superior and inferior vertebrae to stabilize the implant within the intervertebral space. Each bone anchor 100 may extend from a first, trailing end 102 toward a second, leading end 104 configured to enter bone first. The bone anchor 100 includes a head portion 106 and an elongate blade 108 extending therefrom. The head portion 106 may be disposed at the trailing end 102 of the anchor 100 and may have at least a partially spherical outer shape or periphery. In one embodiment, the head portion 106 may be ball shaped or spherical, thereby forming a generally spherical head 106. The spherical head 106 may be generally smooth, without any threads or the like.

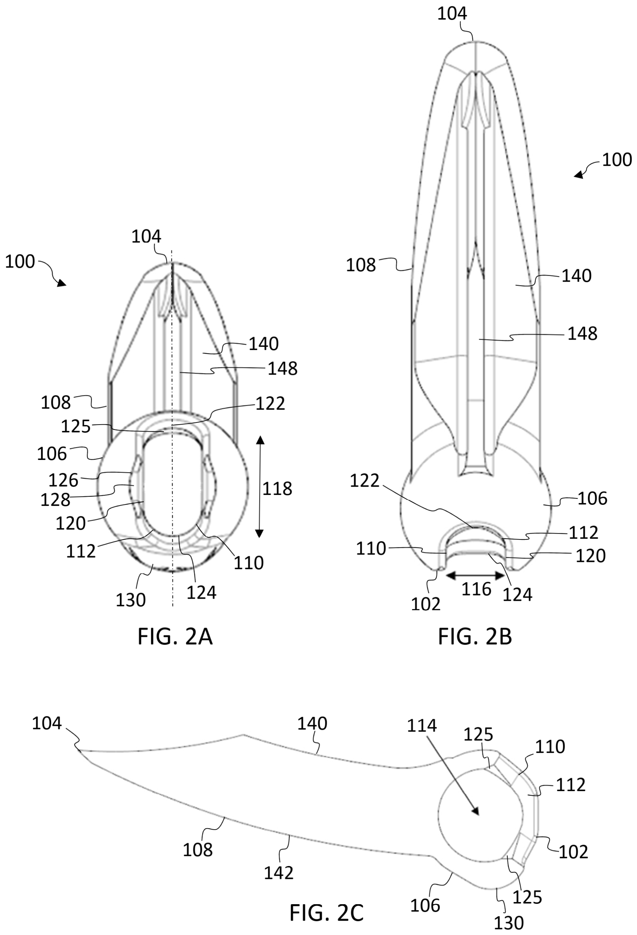

As best seen in FIGS. 2A-2C, the spherical head 106 defines a non-threaded opening 110 configured for receiving a portion of an instrument, such as instrument 200. The non-threaded opening 110 includes a vertical slot 112 in fluid communication with an internal pocket 114 defined within the spherical head 106. When the anchor 100 is oriented with a front view, as shown in FIG. 2A, the vertical slot 112 is centrally located and cuts directly through a front portion of the sphere. The blade 108 extends from a rear portion of the sphere opposite to the vertical slot 112. The slot 112 extends vertically to bisect the spherical surface of the head 106. The vertical slot 112 may align with a longitudinal axis of the anchor 100. The vertical slot 112 may allow for instruments, such as instrument 200, to align in a straight, direct manner with the anchor 100 without angular deviation. In this manner, when engaged with the instrument 200, the anchor 100 is only able to pivot along the axis of the vertical slot 112.

The vertical slot 112 may have a narrow width 116 with an elongated length 118 longer than the width 116. The width 116 may be defined by sides 120 of the slot 112. The sides 120 of slot 112 may be generally straight or planar to maintain a uniform width 116 throughout its length. The length 118 may be defined by ends 122, 124 of the slot 112. In one embodiment, the vertical slot 112 may have a generally obround shape. From front view of FIG. 2A, a first or top end 122 of the slot 112 is located toward a top of the sphere and a second or bottom end 124 of the slot 112 is located toward a bottom of the sphere. It will be appreciated that these positional relationships do not limit the orientation of the anchor in use, which may be provided in any suitable orientation, position, or location as selected by the surgeon. The ends 122, 124 of the slot 112 may include a curved or rounded edge, for example, forming a semicircle or other segment of a circle. The perimeter of the slot 112, especially along ends 122, 124, may be slightly rounded, beveled, or contoured from the outer surface of the sphere toward the slot 112 to improve tool insertion. An under portion of the slot 112 may form an undercut to further engage with instrumentation. In particular, undercuts 125 may be located under each end 122, 124 of the slot 112. The vertical slot 112 may be slightly offset toward the top of the sphere. For example, when the anchor 100 is oriented from the top view shown in FIG. 2B, the top end 122 of slot 112 cuts deeper into the sphere relative to the bottom end 124 of the vertical slot 112. Each side 120 of the slot 112 may further define a lip 126 or protrusion to further mate with the insertion instrument. The lips 236 may include rounded humps or bulges that extend radially outward from the sphere. The lips 126 may include symmetric lips of uniform size and shape on both sides of the slot 112, which do not extend the full length of the slot 112. In one embodiment, the symmetric lips 126 may be located closer toward the bottom end 124 of the slot 112. The apex of the lips 126 may be truncated with a small flat 128 for better connection with the instrumentation.

With further emphasis on FIG. 2C, the vertical slot 112 extends into the spherical head 106 to provide access to the internal pocket 114. The internal pocket 114 forms a cavity defined within the spherical head 106. The internal pocket 114 may include a concave recess, for example, defining a partially spherical, hemispherical, or fully spherical opening or cavity therein. The spherical pocket 114 may be located centrally within the spherical head 106 to maintain structural integrity of the walls of head 106. The configuration of the enlarged spherical cavity 114 relative to the narrow vertical slot 112 creates an overhang along and/or beneath the sides 120 of the slot 112. The overhang creates a lip or barrier, which can prevent instrumentation from unintentionally disengaging from the anchor 100.

The spherical head 106 may also include a bump 130 on its underside to limit the anchor's possible rotation in the channel of the interbody implant once it is inserted. The bump 130 may include a raised, rounded, or convex protrusion. The bump 130 may have a partially spherical or semi-spherical outer surface with a radius smaller than the radius of the spherical head 106. The bump 130 may also align with the longitudinal axis of the anchor 100. As best seen in FIG. 2A, the bump 130 may be located beneath the bottom end 124 of the slot 112 with a symmetrical configuration having a width greater than the width 116 of the slot 112. The bump 130 creates a thickened wall area for the spherical head 106 between the inner pocket 114 and the outer surface of the bump 130. The edges of the bump 130 may blend seamlessly from the outer surface of the sphere with a bevel or contour, for example, to provide for a smooth transition.

The elongate blade 108 extends away from the head portion 106 toward the leading end 104 of the anchor 100. The blade 108 may be curvilinear or have a curvature along the long axis of the blade 108. The blade 108 may be curved such that a concave surface 140 and a convex surface 142 extend from the trailing end 102 toward the leading end 104 of the anchor 100. The leading end 104 may narrow toward a point, bladed edge, or cutting edge, for example, to facilitate impaction and insertion into and through bone. Alternatively or additionally, one or more edges of the blade 108 may further include teeth, tapers, or bevels to further facilitate insertion of the anchor 100 into bone.

The blade 108 may include an elongate fin 148 that extends away from the head portion 106 toward the leading end 104 of the anchor 100. The fin 148 may extend away from the concave surface 140, for example, such that the elongate fin 148 is generally orthogonal to the blade 108. In this manner, the fin 148 may form a generally T-shaped cross-section, which may help to reduce impaction forces and/or increase torsional stability of the anchor 100. It will be appreciated that any suitable blade 108 may be selected to maximize penetration into bone while increasing fixation once installed in the implant.

The anchors 100 may be fully 3D printed to accommodate its unique geometry and to reduce manufacturing costs. Utilizing additive manufacturing, such as laser sintering or fused deposition modeling, the intricate geometry of the spherical pocket 114 and vertical slot 112 may be created. The raised lips 126 on the head 106 of the anchor 100 may also include small flats 128 to avoid overheating of a single point condition in this location. Localized overheating can cause the material to deform or alter the microstructure of the material. Overheating can also cause the anchor to not mate properly to the inserter. The anchor 100 is intended to be able to be fully 3D printed with no post-machining, aside from buffing and/or deburring any remaining support material on the underside of the anchor. Optionally, the anchors 100 may be printed with a roughened 3D printed surface configured to encourage boney on-growth and increase expulsion resistance. The anchors 100 may be provided in a kit in a variety of styles and/or lengths. For example, the anchors 100 may have lengths from spherical center to tip ranging from about 20 to 30mm or other suitable measurements.

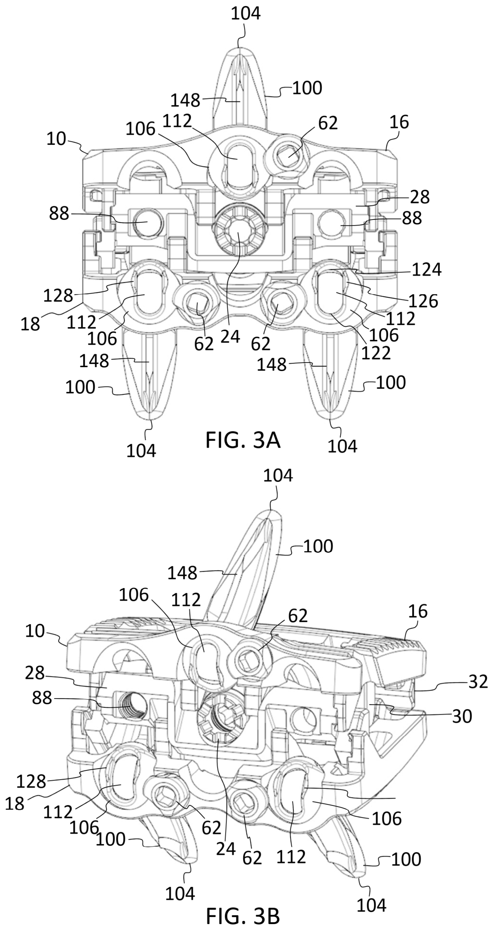

As best seen in FIGS. 3A-3B, the bone anchor(s) 100 may be used in combination with an intervertebral implant, such as expandable implant 10, for example, to form a standalone intervertebral fusion device. In one embodiment, a first bone anchor 100 may be installed through a top portion of the implant 10 and into a superior vertebra while second and third bone anchors 100 may be installed through a bottom portion of the implant 10 and into an inferior vertebra. The bone anchors 100 may be installed through the implant 10 and into adjacent bone to ensure stability and robust attachment to the vertebrae when driven into the vertebral bodies above and below the disc space. Expandable implant 10 may include adjustable lordosis interbody spacers installed from an ALIF (anterior lumbar interbody fusion) approach or other suitable devices. It will be appreciated, however, that the number, location, and trajectory of the anchors 100 may be modified and may be used with other suitable devices for optimal fixation.

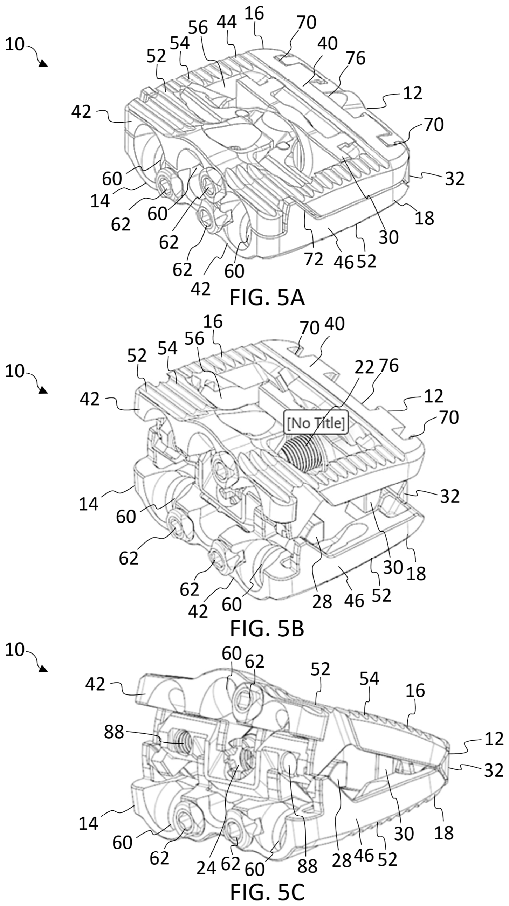

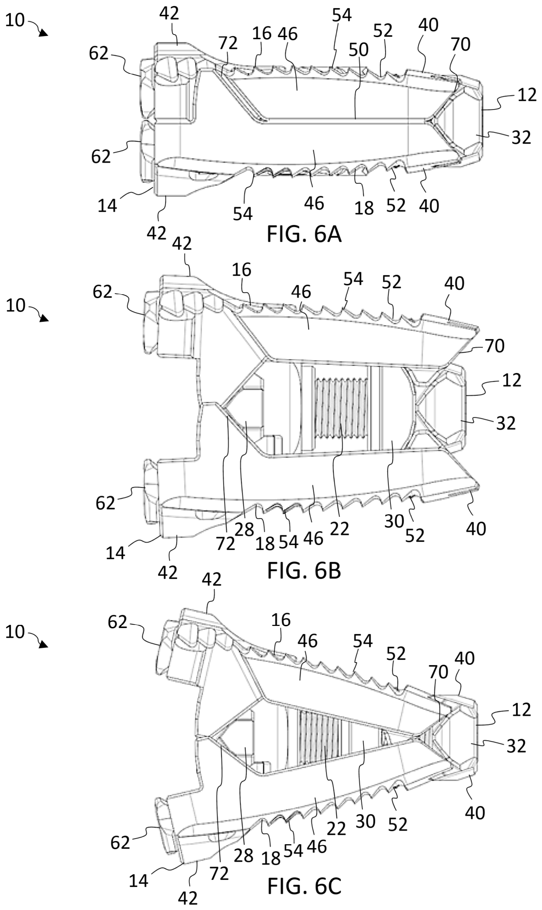

With further emphasis on FIGS. 5A-5C and 6A-6C, the expandable implant 10 extends along a central longitudinal axis between a front end or posterior end 12 and a rear end or anterior end 14 of the device 10. The implant 10 includes upper and lower endplates 16, 18 configured to engage adjacent vertebrae, which define a height of the implant 10. The implant 10 may include an actuator assembly configured for expanding the upper and lower endplates 16, 18. The actuator assembly may include a single actuator screw 22 threaded into anterior and posterior actuator ramps 28, 30 to independently control the anterior and posterior height of the implant 10.

In one embodiment, the expandable implant 10 may include three separate actuators 28, 30, 32 positioned between the upper and lower endplates 16, 18: a moveable anterior actuator 28, a moveable posterior actuator 30, and a stationary posterior actuator 32. One end of the actuator screw 22 threads into an anterior actuator nut 24 located in the anterior actuator 28 and the opposite end of the actuator screw 22 threads into a posterior actuator nut affixed to the stationary posterior actuator 32. The actuator screw 22 threads through the moveable posterior actuator 30 to translate the posterior actuator 30 along central longitudinal axis. When the actuator screw 22 and anterior actuator nut 24 are turned together, the moveable posterior actuator 30 moves toward the stationary posterior actuator 32, forcing the upper and lower endplates 16, 18 apart resulting in parallel expansion. When only the anterior actuator nut 24 is turned, the moveable anterior actuator 28 moves alone to increase the anterior height, resulting in an increase in lordotic angle.

The upper and lower endplates 16, 18 interface with the actuators 28, 30, 32 via a plurality of mating ramps. The complimentary mating ramps may include angled, inclined, or sloped surfaces that slidably interface with one another to allow for expansion of the upper and lower endplates 16, 18. The mating ramps allow for controlled, sliding movement on the complementary surfaces, thereby providing vertical displacement of endplates 16, 18.

The implant 10 is configured to be inserted into the disc space in a collapsed configuration. FIG. 5A shows the implant 10 in the fully collapsed configuration. Once inserted into the disc space, the implant 10 is expanded in height to an expanded configuration to precisely restore spinal alignment and distribute load across the vertebral endplates. FIG. 5B shows the implant 10 in a fully expanded configuration with maximum expansion. FIG. 5C shows the implant 10 in an expanded configuration with maximum lordosis. It will be appreciated that the implant 10 may be expanded to any desired height between these extremes. The anterior and posterior heights are independently adjustable to a desired lordotic profile. In this manner, the height is adjustable to restore height loss in the disc space and lordotic angulation. It should be understood that reference to the front and rear ends and anterior and posterior heights are described with respect to the direction of placement into an intervertebral disc space with the front of the expandable fusion device 10 placed into the disc space first, followed by the rear of the expandable fusion device 10, and then expanding the endplates 16, 18 in height and/or lordosis. These and other directional terms may be used herein for descriptive purposes and do not limit the orientation(s) in which the devices may be used.

Each endplate 16, 18 may include a front or posterior rail 40 and a rear or anterior rail 42 extending between opposed side rails 44, 46. The rails 40-46 define an inner face 50 and an opposite outer face 52. The inner face 50 may be configured to mate with the respective actuators 28, 30, 32 and the outer face 52 may be configured to contact adjacent vertebrae. The outer face 52 of each endplate 16, 18 may include a plurality of teeth 54 or other friction increasing elements, such as ridges, roughened surfaces, keels, gripping or purchasing projections configured to retain the device 10 in the disc space. The endplates 16, 18 may be 3D printed using additive manufacturing. In this manner, the outer face 52 may be created with teeth and/or surface texturing that can better facilitate bony on-growth. Each endplate 16, 18 may define a vertical window or through passage 56, thereby defining a central graft chamber within the implant 10. The window or through passage 56 allows graft material or other therapeutically beneficial material to be packed into or grow through the implant 10.

The implant 10 may be secured to the adjacent vertebrae with one or more fixation screws and/or anchors, such as anchors 100. For example, the anterior rail 42 may define at least one anchor socket 60 configured for receiving the anchor 100 therethrough and into the adjacent vertebra. In the embodiment shown, three sockets 60 for receiving three respective anchors 100 are provided in the upper and lower endplates 16, 18: one socket 60 pointed upward into the superior vertebra and two sockets 60 pointed downward into the inferior vertebra. The sockets 60 may be surrounded by a partial spherical cut or hemispherical recess such that the anchors 100 may be angled into the adjacent vertebrae. The partial spherical cut of the socket 60 provides a congruent surface for the spherical head 106 of the anchor 100 to permit rotation of the anchor 100 within the socket 60. The sockets 60 in the endplates 16, 18 may be compatible with both traditional lag screws and/or anchor fixation, such as anchors 100. In other words, the sockets 60 function as bone screw holes and/or guides for the anchors 100. The diameter of the anchor head 106 may match that of bone screws, allowing either anchors 100 or screws to be inserted into each of the bone screws holes or sockets 60 on the interbody. Examples of bone screws and anchors are further described in in U.S. Patent No. 11,554,023, which is incorporated by reference herein in its entirety for all purposes. Although a given configuration of sockets 60 is shown, it will be appreciated that the sockets 60 may be present in any suitable number, location, and configuration for fixation.

Cam style blocking screws 62 may be used to block the anchors 100 (or bone screws) from backing out after being inserted. The anterior rail 42 may define a blocking screw hole positioned next to each respective socket 60. The blocking screw holes may be internally threaded to receive the respective threaded blocking screws 62. In one embodiment, three blocking screws 62 thread into the endplates 16, 18 to secure the anchors 100 in the three respective sockets 60. The blocking screws 62 may have an enlarged head with a drive recess and a threaded shaft. These blocking screws 62 allow for one type of blocking screw to be used across all of the implant sockets 60. When the blocking screws 62 are rotated and engaged, a portion of the enlarged head covers the respective anchors 100, thereby preventing migration of the installed anchors 100.

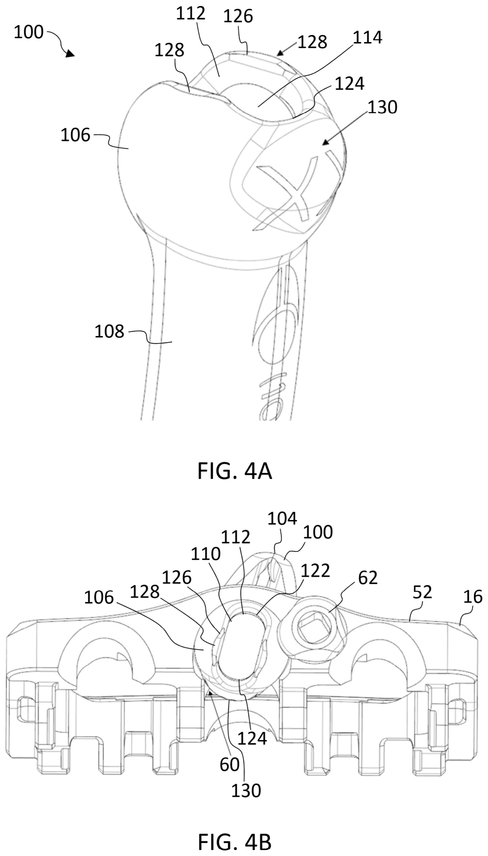

In one embodiment shown in FIG. 4B, the anchor 100 is shown in more detail installed through the upper endplate 16. The anchor head 106 includes bump 130 on the underside to limit the anchor's possible rotation in the socket 60 of the interbody once it is inserted. If the anchor 100 were to have a fully spherical head (without a bump), the anchor would be able to rotate to a position where the vertical slot 112 could line up with the blocking screw 62, creating a situation where the anchor is not protected from back-out. The anti-rotation bump 130 limits rotation of the anchor 100 to prevent this situation. For example, the anti-rotation bump 130 may limit rotation to 20 degrees in either direction where the bump 130 interferes with the endplate geometry. The sides of the bump 130 can abut against a ledge, limit, or other stop in the socket 60, which prevents further turning or rotation of the anchor 100. The 20 degrees of rotation for anchor 100 allows for some variability during insertion, but prevents the slot 112 from lining up with the blocking screw 62. In this manner, the blocking screw 62 prevents the anchor 100 from unintentionally backing out from its seated position within the implant.

The rails 40-46 and/or inner face 50 of each endplate 16, 18 may define one or more ramps 70, 72 configured to mate with the respective actuators 28, 30, 32, thereby causing endplates 16, 18 to separate apart. For example, the endplates 16, 18 may define posterior ramps 70 along the posterior rail 40, which mate with the posterior actuators 30, 32. The endplates 16, 18 define anterior ramps 72 along the anterior rail 42 and central ramps along the side rails 44, 46, which mate with the anterior actuator 28. The ramps 70, 72 may include ramped surfaces, angled surfaces, or inclined planes with a given gradient or angle of slope. The ramps 70, 72 may have generally straight ramped surfaces, may be curved, or may be configured in any suitable manner for slidable interface between the components. The ramps 70, 72 may define male or female slide ramps configured to mate with corresponding ramps on the actuators 28, 30, 32.

The actuator assembly includes moveable anterior actuator 28, moveable posterior actuator 30, and stationary posterior actuator 32 positioned between the upper and lower endplates 16, 18, thereby providing expansion to the implant 10. The anterior actuator 28 may define recesses or bores 88, such as threaded and non-threaded attachment holes 88 designed to interface with instrumentation for insertion and/or removal of the implant. The actuators 28, 30, 32 include one or more ramps configured to mate with corresponding ramps 70, 72 on the endplates 16, 18. The implant 10 allows for continuous expansion and distraction over the range of that specific implant. This provides the ability to distract vertebral bodies to a desired height, but also collapse the device 10 for repositioning if desired. The implant 10 has the ability for the endplates 16, 18 to converge providing lordosis, while maintaining a large window for bone graft placement. By changing lordotic angulation, the implant 10 may match the patient's natural lordosis or be used to provide a specific lordosis at the level(s) treated.

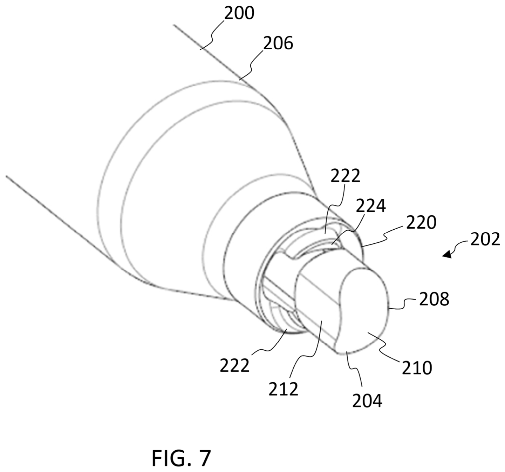

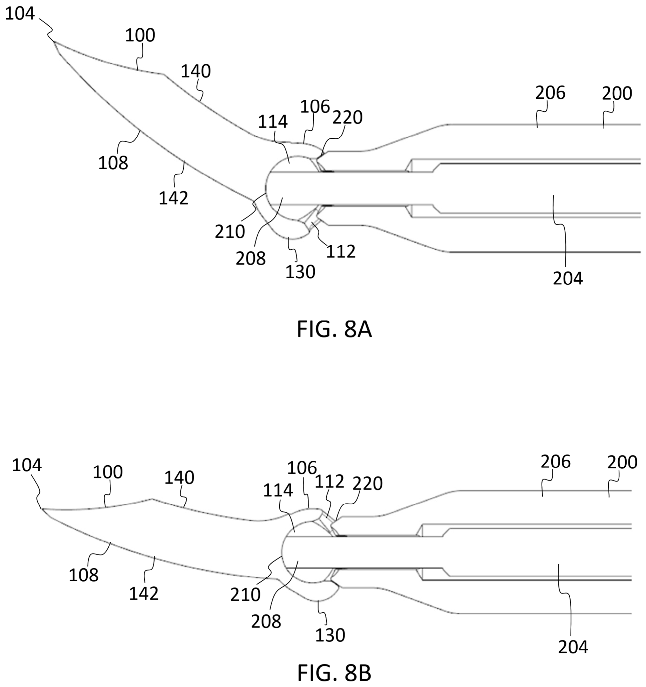

Once implant 10 is installed in the disc space, anchors 100 may be installed to anchor the implant to bone. Turning now to FIGS. 7 and 8A-8B, the anchors 100 are compatible with both straight and angled insertion and removal via instrument 200. Instrument 200 includes a distal tip 202 receivable in the non-threaded opening 110 of the anchor 100 and configured to secure the anchor 100 to the instrument 200. The distal tip 202 is configured to rotate the anchor 100 such that the anchor 100 locks to the instrument 200 for insertion and/or removal. Previous anchor designs have used a threaded connection with the instrumentation, which does not allow for insertion/removal at an angle. Furthermore, it can be very difficult to attach to these connection threads in-situ. As such, the non-threaded interface between instrument 200 and anchor 100 provides for an improved in-situ attachment method for anchor 100. The non-threaded connection engages seamlessly with the inserter tip 202 significantly enhancing the ease of attachment, insertion, and/or removal of the anchor, thereby streamlining the surgical process.

The instrument 200 may include an inner shaft 204 positionable through an outer sleeve 206. The inner shaft 204 includes an elongated tip 208 configured to fit within the vertical slot 112 in the anchor 100. The elongated tip 208 may have a partially rounded or spherical tip with a truncated distal-most end 210. The elongated tip 208 may have rounded or curved sides with at least one flat side 212 configured to mate with the corresponding flat side 120 of the vertical slot 112. The truncated end 210 may have a slight radius of curvature matching the radius of the inner cavity 114. The outer sleeve 206 may include a distal end 220 with a reduced diameter. The distal-most face of end 220 may include one or more protruding tabs or rims 222, which are configured to secure the instrument 200 to the anchor 100 when the anchor 100 is rotated. The rims 222 may follow the shape of an arc or curvature mimicking the shape of the ends 122, 124 of the slot 112 in anchor 100. The rims 222 may have a flat or planar surface 224 as the distal-most surface. The flat surface 224 may be fully exposed when the elongated tip 208 is rotated 90 degrees. Each rim 222 may have a curved surface congruent with the curvature of the inner spherical pocket 114 of the anchor 100. The protruding rims 222 may include a first rim portion and a second rim portion on opposite sides of the elongated tip 208 of the inner shaft 204. When the anchor 100 is rotated with the inner shaft 204, the outer sleeve 206 remains stationary, thereby allowing the protruding rim(s) 222 to hook under the sides 120 of the vertical slot 112 of the anchor 100 functioning similar to a cam lock. The rims 222 may also act as counter-torque to prevent the anchor from rotating in the bone, creating a windshield wiper effect often seen with threaded anchors, which can ruin the boney purchase of the anchor.

As shown in the configuration depicted in FIG. 7, the elongated tip 208 and protruding rims 222 are aligned with one another and sized and dimensioned to fit within the vertical slot 112 of the anchor. The anchor 100 has internal spherical pocket 114 inside the spherical head 106 that can be accessed via vertical slot 112 on the front of the anchor head 106. The inner elongated tip 208 extends into the inner pocket 114 of the anchor 100 and the flats 212, 120 align with one another. The truncated end 210 of tip 208 may bottom out within the pocket 114 (e.g., as shown in FIGS. 8A-8B). The vertical slot 112 allows spherical tip 208 with flat side(s) 212 to be inserted into the internal spherical pocket 114 and then the inner shaft 204 is rotated about the long axis of the instrument 200 to securely engage the anchor 100 to the instrument 200. For example, the inner shaft 204 may rotate the anchor 100 about 90 degrees or a quarter-turn to securely engage the anchor 100 to the instrument 200. In this manner, the inner shaft 204 rotates 90 degrees, thereby also rotating the anchor 90 degrees about the long axis of the instrument 200 while the outer sleeve 206 remains stationary. The rotation of anchor 100 causes the rim(s) 222 of the outer sleeve 206 to fit under the overhang along the sides 120 of the vertical slot 112, thereby functioning in a manner similar to a cam lock. As best seen in FIG. 8A-8B, when engaged with the instrument 200, the length 118 of the slot 112 also permits the anchor 100 to pivot along the axis of the vertical slot 112. The slot 112 permits the anchor 100 to pivot up to 22 degrees on the inserter tip 202. This variable angle allows for navigation around difficult anatomy through a small surgical incision. The variable angle for anchor 100 may be especially helpful during insertion, for example, when faced with a steep sacral slope at L5/S1.

The vertical slot 112 also doubles as an anti-rotation feature to counter-torque the anchor 100 to the inserter 200 while the inner spherical tip 208 is rotated 90 degrees inside the anchor head 106 to attach or detach from the anchor 100. Previous anchor designs have used a threaded connection. In instances of overtightening or poor bone quality, attempting to loosen the inserter from the anchor by rotating these threads has caused the anchor to rotate in the bone or windshield wiper, ruining the boney purchase of the anchor. The quarter-turn anchor attachment method provides an improvement over the threaded connection in existing anchors while also providing navigation for straight and angled insertion and/or removal of the anchors 100. The internal spherical pocket 114 also creates a surface that can be used to apply force to remove the anchor 100 with the same instrument 200 that was used for insertion.

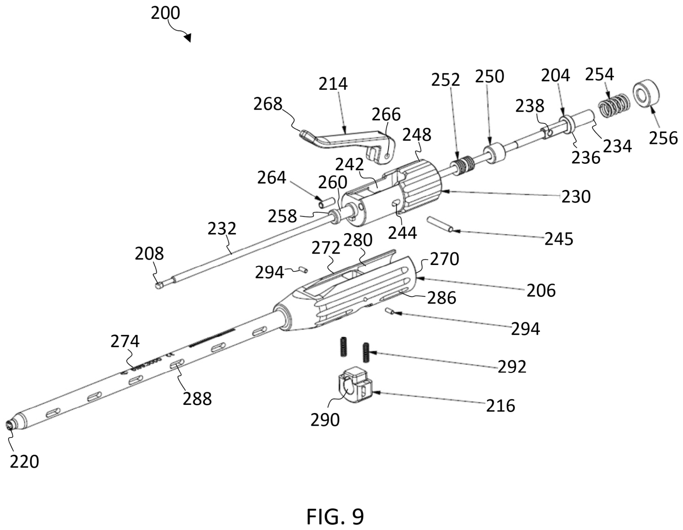

Turning now to FIG. 9, an exploded view of single anchor impactor instrument 200 is shown according to one embodiment. In one embodiment, the instrument 200 is configured to insert and/or remove anchors 100 into the sockets 60 of the adjustable lordosis interbody spacer 10. Unlike other instruments inserting anchors with an anchor guide and/or impactor rods, instrument 200 is configured to install each anchor 100 freehand while the socket 60 of the implant 10 acts as a guide surface for the anchor 100. The single anchor impactor 200 is able to quickly and easily attach to the anchor 100 at a straight-on or angled trajectory, maintain a secure connection with the anchor 100 through impaction, and quickly release the anchor 100 once properly positioned. The anchor impactor 200 may also be used as a removal tool to pull anchors 100 out of the bone and/or interbody spacer if necessary. The single anchor impactor instrument 200 may include inner shaft 204 and outer sleeve 206 aligned along the instrument axis, a lever 214 for locking the anchor 100 to the instrument 200, and a release button 216 for disassembling the instrument components.

The inner shaft 204 includes a rear housing 230 receiving a central shaft 232 therethrough. The central shaft 232 may have a generally cylindrical body, which is positioned through the rear housing 230. The central shaft 232 extends from proximal end 234 and terminates as distal tip 208 configured to engage with anchor 100. The central shaft 232 may include an enlarged shoulder 236 near the proximal end 234, which is configured for retaining spring 254 and a through opening 238 for securing the lever 214 to the central shaft 232 via pin 245. The rear housing 230 may have a generally cylindrical body defining a central longitudinal opening 240 sized and dimensioned to receive the central shaft 232. The rear housing 230 may define a channel 242 configured to receive a portion of the lever 214, a transverse pin opening 244 for pinning the lever 214 to the rear housing 230 via pin 245, and a transverse button opening 246 for receiving release button 216. An outer surface 248 of the rear housing 230 may include a grip, such as longitudinal slits/protrusions, which provides a surface for the user to rotate the inner shaft 204 relative to the outer sleeve 206.

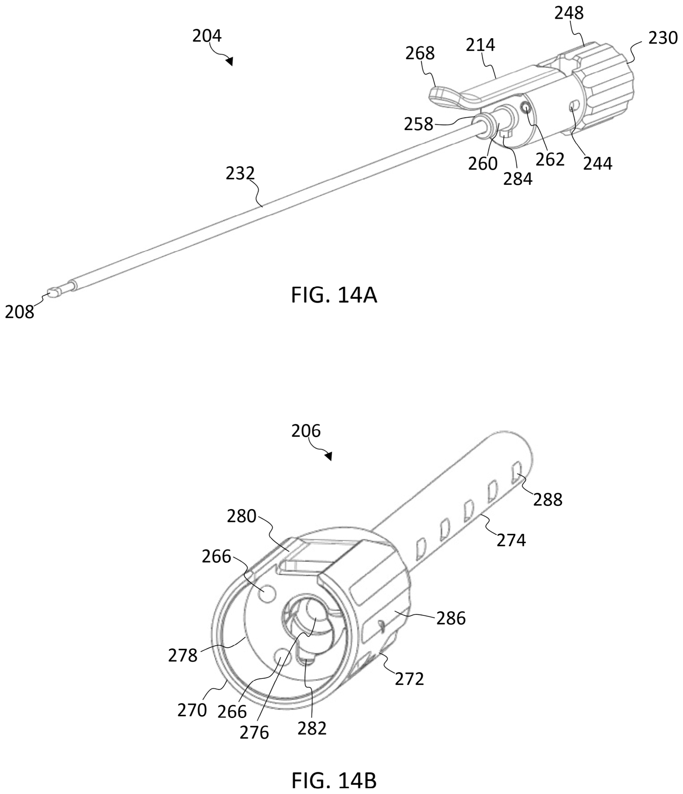

The central shaft 232 retains a shuttle 250 and multiple washers 252. The shuttle 250 may include a tubular body positioned on the central shaft 232 within the rear housing 230. The washers 252 may include spring washers, such as Belleville washers, positioned on the central shaft 232 within rear housing 230. During assembly, the shuttle 250 and multiple Belleville washers 252 slide over the central shaft 232. The central shaft 232 is inserted into the rear housing 230. The lever 214 is pinned via pin 245 to the central shaft 232 inside the rear housing 230 of the instrument 200. The shuttle 250 and washers 252 are configured to slide axially over the central shaft 232 when the lever 214 is actuated and the central shaft 232 is moved axially. The washers 252 may be located between the release button 216 and the shuttle 250, while the shuttle 250 is positioned between the washers 252 and the lever pin 245. The spring 254 is positioned around the central shaft 232 toward the proximal end 234 of the shaft 232. The spring 254 may be retained by a cap 256, which fits inside the rear housing 230. The cap 256 may be welded to the rear housing 230 to permanently capture the spring 254 therein. As best seen in FIG. 14A, the central shaft 232 may include a shoulder 258 beneath the rear housing 230, which defines an engagement area 260 for the release button 216. The base of the rear housing 230 may further define an opening 262 configured to receive one end of a ball detent 264, such as a ball plunger. The other end of the ball detent 264 may protrude from the rear housing 230 such that the detent 264 is configured to interface with divots 266 in the housing 272 of the outer sleeve 206. For example, two divots 266 may be provided in housing 272 of the outer sleeve 206 to hold the inner shaft 204 in one of two relative positions rotated 90 degrees apart from one another.

The anchor 100 may be locked to instrument 200 via lever 214. The lever 214 includes an elongate arm pivotable about pin 245, which acts a pivot axis. The central shaft 232 is positionable through the lever 214, and the lever 214 positioned in channel 242 in the rear housing 230. The pin 245 is receivable through opening 244 in the rear housing 230, through opening 266 in the lever 214, and through opening 238 in the central shaft 232, thereby forming the pivot axis for lever 214. On the opposite end, the lever 214 terminates at free end 268. In the open position, the free end 268 of the lever 214 is pivoted away from the body of the rear housing 230, and the lever 214 may be rotated when the inner shaft 204 is rotated relative to the outer sleeve 206. In the open and unlocked positions, the lever 214 may be aligned generally orthogonal to the long axis of the instrument 200. In a locked position, the lever 214 is pivoted flush to the body of the rear housing 230. In the locked position, the lever 214 may be aligned generally parallel to the long axis of the instrument 200. The free end 268 may be splayed slightly outward so the user can grasp the lever 214 to pivot it out from the rear housing 230 to return to the unlocked position.

The outer sleeve 206 extends from a proximal end 270 to distal end 220. The outer sleeve 206 may include a housing portion 272 and a tubular body 274 extending therefrom. The outer sleeve 206 defines a central through opening 276 sized and dimensioned to receive the inner shaft 204 therethrough. The housing portion 272 may include a recessed area 278 at the proximal end 270, which receives a portion of the rear housing 230 of the inner shaft 204. For example, the rear housing 230 may fit inside the housing portion 272 such that the outer grip 248 of the inner shaft 204 remains accessible to the user for rotation. A side of the housing portion 272 of the outer sleeve 206 may define a channel 280 configured to receive a portion of the lever 214 when the inner shaft 204 is seated in the outer sleeve 206 and the lever 214 is actuated into the locked position. As best seen in FIG. 14B, the outer sleeve 206 may define an alignment recess 282 configured to receive a corresponding alignment key 284, such as a keying tab. The alignment recess 282 may include a cutout in communication with the central through opening 276 sized and dimensioned to receive keying tab 284 on an underside of the rear housing 230. The keying tab 284 may be seated into the keying recess 282 to ensure the lever 214 is properly aligned during assembly. An outer surface 286 of the housing 272 may include a grip, such as longitudinal slits/protrusions, which provides a surface for the user to hold the outer sleeve 206, for example, when rotating the inner shaft 204 and holding the outer sleeve 206 stationary. The tubular body 274 of the outer sleeve 206 may also define one or more windows 288, for example, to facilitate visualization and/or cleaning.

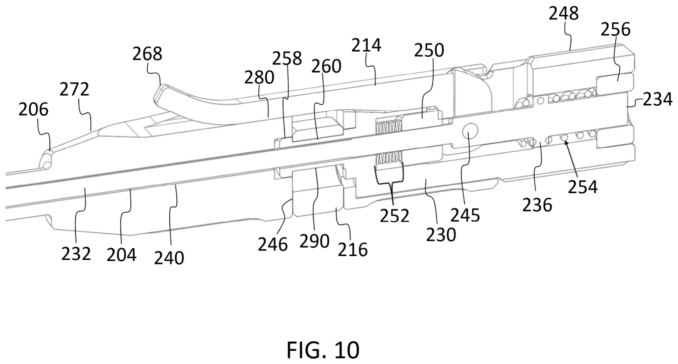

As best seen in FIG. 10, the inner shaft 204 may be released from the outer sleeve 206 via release button 216. The release button 216 may define a through opening 290 which receives central shaft 232 therethrough. The release button 216 may be spring-loaded via springs 292. The button 216 may be pinned to the outer sleeve 206 via pins 294. When in the locked position, the button 216 is biased against the engagement surface 260 of the central shaft 232 to snugly engage and lock the outer sleeve 206 to the inner shaft 204. When the button 216 is depressed into an unlocked position, the button 216 releases from the engagement surface 260, thereby permitting the inner shaft 204 and outer sleeve 206 to be disassembled from one another, for example, to clean the instrument parts.

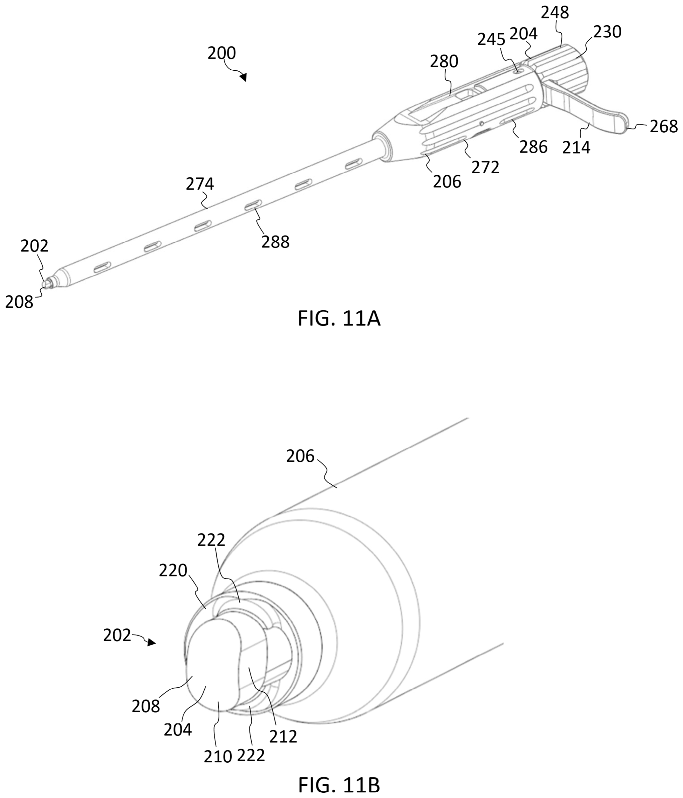

The single anchor impactor 200 may function in three main positions: (1) instrument 200 is ready for attachment to the anchor 100; (2) anchor 100 is attached to the instrument 200 in an unlocked position; and (3) anchor 100 is attached to the instrument 200 in a locked position. In the first position, the anchor 100 is ready for attachment to the instrument 200. As best seen in FIGS. 11A-11B, the impactor instrument 200 is configured to attach to the anchor 100. The lever 214 is extended outward and oriented in a first position such that the lever 214 is generally orthogonal to the long axis of the instrument 200. The tip 208 of the inner shaft 204 is oriented to fit into the vertical slot 112 in the anchor 100. The flat side 212 of the tip 208 is configured to match with the corresponding flat side 120 of the vertical slot 112. The rims 222 of the outer sleeve end 220 also fit into the ends 122, 124 of the vertical slot 112 in anchor 100. In this ready orientation, the spherical tip 208 of the inner shaft 204 may be fully inserted into the anchor head 106.

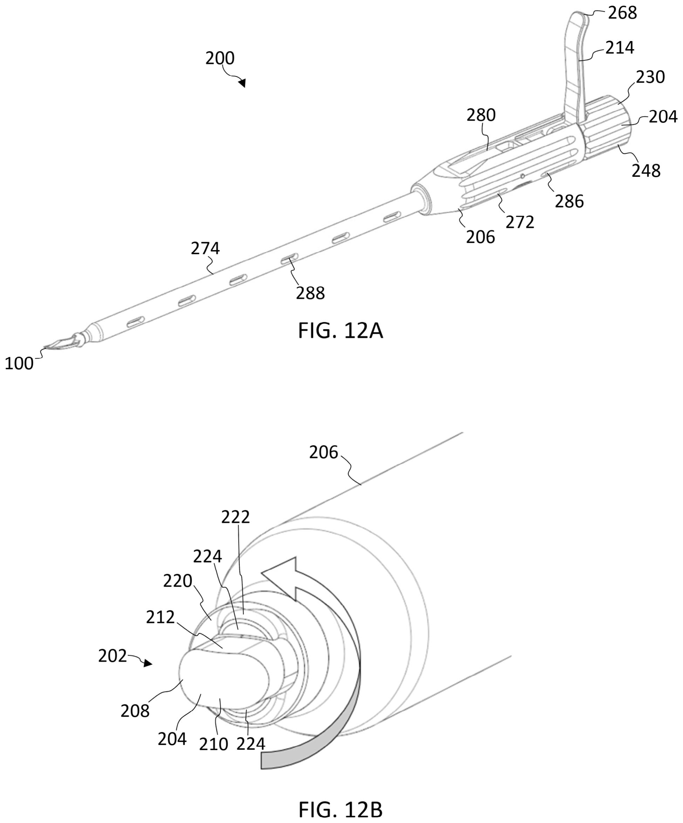

In the second position, the anchor 100 is attached to the instrument 200 but not locked. As best seen in FIGS. 12A-12B, the lever 214 is still extended orthogonally but rotated 90 degrees relative to its first position. Accordingly, the inner shaft 204 is also rotated 90 degrees by turning the lever 214 into this second position. This causes the distal tip 208 to rotate 90 degrees, thereby rotating anchor 100 likewise 90 degrees while the outer sleeve 206 remains stationary. The rotated position of distal tip 208 may also expose the flat surfaces 224 of rims 222. As the anchor 100 is rotated relative to the stationary outer sleeve 206, the rims 222 hook under the sides 120 of the slot 112. This prevents the anchor 100 from being removed from the instrument 200, but anchor angulation can still be adjusted.

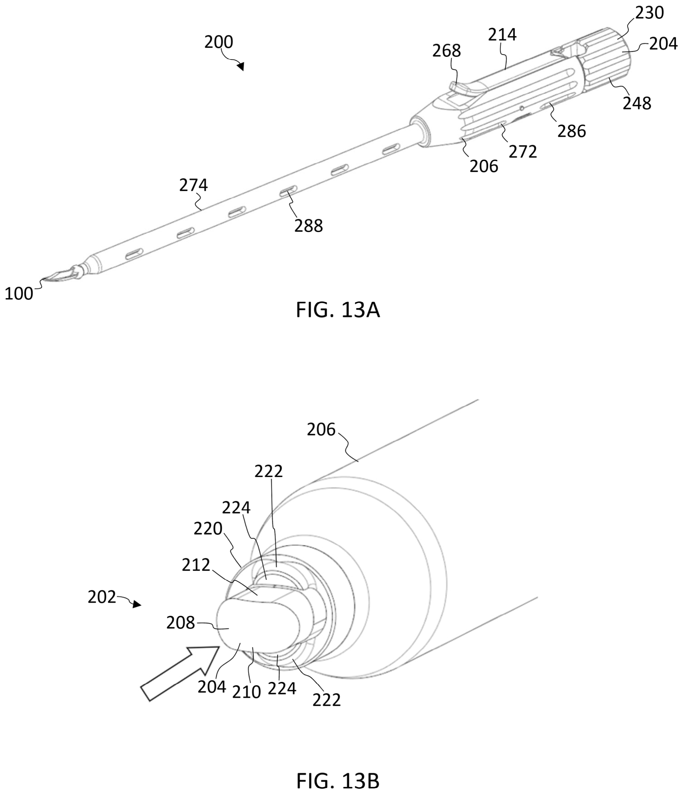

In the third position, the anchor 100 is attached and locked to the instrument 200. As best seen in FIGS. 13A-13B, the lever 214 is pivoted flush against the body 272 of the outer sleeve 206. The central shaft 232 is pulled back by lowering the lever 214. This compresses the Belleville washers 252, holding the anchor 100 tight against the body of the instrument 200. When the lever 214 is lowered, it pulls the inner shaft 232 backwards toward the proximal end. The backwards axial motion of the central shaft 232 is stopped when the spherical tip 208 of the central shaft 232 pulls the anchor 100 tight to the body (e.g., end 220) of the instrument 200. At this point, the lever 214 is able to finish being fully depressed by pushing the shuttle 250 forward, compressing the washers 252. The compression of the washers 252 keeps tension on the central shaft 232 pulling the anchor 100 tight to the distal end 220 of the outer sleeve 206.

Once locked to the instrument 200, the anchor 100 can be installed freehand through the implant 10 and into bone. Each bone anchor 100 may be inserted through the respective sockets 60 in the implant 10, which function as guide channels to help guide the anchors 100 into the adjacent bone. An impaction force can be applied to the proximal end of the instrument 200, which is transmitted to the anchor 100 to drive the anchor 100 into the precise location within the bone. After installation, the instrument 200 can be unlocked and removed from the anchor.

The anchor 100 may be unlocked by releasing the lever 214. When the lever 214 is raised, the central shaft 232 is allowed to move forward, loosening the pull on the anchor 100. The return spring 254 ensures the central shaft 232 is fully pushed forward. The lever 214 must be in the proper orientation with the rear housing 230 in order to execute the third position of locking the anchor 100 to the inserter 200. This ensures the inner shaft 204 is properly engaged with the anchor 100, before pulling it tight. In a similar way, the inner shaft 230 cannot be rotated to release the anchor 100 until the lever 214 is lifted fully vertical. This is a safety feature to prevent accidental release of the anchor 100.

The rear housing assembly may include ball detent 264 that interfaces with divots 266 in the housing 272 to hold the inner shaft 204 in one of the two relative positions at 90 degrees apart: the ready to attach position from the first position, and the attached position in the second and third positions. This provides tactile feedback to the user that the inner shaft 204 is rotated to the correct position. The rear housing 230 may also include keying tab 284 that prevents the rear housing assembly 230 from being inserted into the body 272 of the instrument 200 in the wrong orientation.

The anchor impactor instrument 200 may be configured to easily disassemble for cleaning purposes or storage. The inner shaft 232 and rear housing assembly 230 may be removed from the body 272 of the instrument 200 by depressing the release button 216 on the body of the instrument 200. Springs 292 return the button 216 to the locked state, and pins 294 keep the button 216 retained in the instrument body.

The anchor impactor provides a quick and intuitive quarter-turn connection to a single anchor. Tabs on inserter tip counter-torque the anchor to prevent the windshield wiper effect often seen with threaded anchors. The spherical attachment tip allows for insertion and/or removal from 0 to 22 degrees of angulation, accommodating difficult anatomy that is an obstacle for threaded anchors. The same instrument can be used to insert and remove the anchors. The lever and compression washer design holds the anchor securely to the inserter, avoiding damage to the instrument tip. The lever design also ensures that the inner shaft cannot be accidentally turned to disengage the anchor. The ergonomics of the design ensure the users hand holds the lever down during impaction. The instrument easily disassembles into manageable components for cleaning.

Although the invention has been described in detail and with reference to specific embodiments, it will be apparent to one skilled in the art that various changes and modifications can be made without departing from the spirit and scope of the invention. Thus, it is intended that the invention covers the modifications and variations of this invention provided they come within the scope of the appended claims and their equivalents. It is expressly intended, for example, that all components of the various devices disclosed above may be combined or modified in any suitable configuration.

The invention could be defined inter alia by the following examples:
1. A bone anchor for securing an intervertebral implant comprising: a spherical head and a curved blade extending therefrom, the spherical head defining a non-threaded opening including a vertical slot in communication with an internal spherical pocket defined within the spherical head, the vertical slot has a narrow width defined between opposing flat sides with an elongated length longer than the width, the vertical slot being aligned with a longitudinal axis of the anchor, the spherical head includes a raised bump located beneath the vertical slot creating a thickened wall area, the raised bump having a radius smaller than the radius of the spherical head and a width greater than the width of the vertical slot.
2. The anchor of Example 1, wherein the vertical slot has an obround shape.
3. The anchor of Example 1, wherein the vertical slot is offset toward a top of the spherical head such that a top end of the vertical slot cuts deeper into the spherical head relative to a bottom end of the vertical slot.
4. The anchor of Example 1, wherein the internal spherical pocket has a diameter greater than the width of the vertical slot, thereby forming an overhang beneath the sides of the vertical slot.
5. The anchor of Example 1, wherein each side of the vertical slot defines symmetric lips.
6. The anchor of Example 5, wherein an apex of each lip is truncated with a flat.
7. The anchor of Example 1, wherein the curved blade includes an elongate fin that ends away from a concave surface of the blade such that the elongate fin is generally orthogonal to the blade.
8. A spine stabilization system comprising: an expandable implant having an upper endplate, a lower endplate, and an actuator assembly including a moveable anterior actuator, a moveable posterior actuator, and a stationary posterior actuator positioned between the upper and lower endplates to independently control anterior and posterior heights of the implant; a bone anchor insertable through the upper or lower endplate and into adjacent bone, the bone anchor having a spherical head and a curved blade extending therefrom, the spherical head defining a non-threaded opening including a vertical slot in communication with an internal spherical pocket defined within the spherical head, the vertical slot has a narrow width defined between opposing flat sides with an elongated length longer than the width, the vertical slot being aligned with a longitudinal axis of the anchor, the spherical head includes a raised bump located beneath the vertical slot creating a thickened wall area, the raised bump having a radius smaller than the radius of the spherical head and a width greater than the width of the slot.
9. The spine stabilization system of Example 8, wherein the upper and/or lower endplates define a socket configured to receive the spherical head of the bone anchor, which guides the anchor into the adjacent bone.
10. The spine stabilization system of Example 9, wherein the socket has a partial spherical cut, which provides a congruent surface for the spherical head of the anchor to permit rotation of the anchor within the socket.
11. The spine stabilization system of Example 9, wherein the socket defines a stop, which engages with the raised bump to limit rotation of the anchor.
12. The spine stabilization system of Example 11, wherein the stop allows for up to 20 degrees of rotation for the anchor within the socket.
13. The spine stabilization system of Example 8, wherein the expandable implant includes a blocking screw, which when rotated, covers a portion of the spherical head of the anchor, thereby preventing the anchor from backing out after insertion, and the raised bump prevents the non-threaded opening of the anchor from aligning with the blocking screw.
14. A method of implanting an orthopedic implant comprising: inserting an orthopedic implant between adjacent vertebrae, the orthopedic implant comprising a first endplate defining a first socket, a second endplate defining a second socket, and an actuator assembly including a moveable anterior actuator, a moveable posterior actuator, and a stationary posterior actuator positioned between the upper and lower endplates to independently control anterior and posterior heights of the implant; expanding the implant to adjust height and/or lordosis; attaching a first anchor to a single anchor impactor instrument having a non-threaded distal tip configured to mate with a non-threaded opening in the anchor, the instrument includes an inner shaft positioned through an outer sleeve, the inner shaft includes an elongated tip having curved sides with at least one flat side configured to mate with a corresponding flat side within a vertical slot of the anchor, the outer sleeve includes a distal end with a protruding rim, wherein the first anchor is attached to the instrument by rotating the inner shaft, which rotates the anchor such that the protruding rim of the outer sleeve fits under an overhang along sides of the vertical slot, thereby securing the first anchor to the instrument; pivoting the first anchor along an axis of the vertical slot to accommodate a straight or angled trajectory for the first anchor; and inserting the first anchor into the first socket of the first endplate to engage an upper vertebra.
15. The method of Example 14, wherein the inner shaft includes a ball detent configured to engage with divots within a housing of the outer sleeve such that the inner shaft is held in one of two relative positions defined by the divots when the inner shaft is rotated with respect to the outer sleeve.
16. The method of Example 14, wherein the inner shaft rotates 90 degrees relative to the stationary outer sleeve.
17. The method of Example 14, further comprising a length of the vertical slot permits the anchor to pivot based on limits imposed by ends of the vertical slot.
18. The method of Example 14, further comprising attaching a second anchor to the single anchor impactor instrument by rotating the inner shaft and the anchor such that the protruding rim of the outer sleeve fits under the overhang along the sides of the vertical slot, thereby securing the second anchor to the instrument.
19. The method of Example 18, further comprising inserting the second anchor into the second socket of the second endplate to engage a lower vertebra.
20. The method of Example 14, further comprising disassembling the inner shaft from the outer sleeve by depressing a release button within the outer sleeve to disengage from the central shaft.

## Claims

1. A bone anchor for securing an intervertebral implant comprising:
a spherical head and a curved blade extending therefrom, the spherical head defining a non-threaded opening including a vertical slot in communication with an internal spherical pocket defined within the spherical head, the vertical slot has a narrow width defined between opposing flat sides with an elongated length longer than the width, the vertical slot being aligned with a longitudinal axis of the anchor, the spherical head includes a raised bump located beneath the vertical slot creating a thickened wall area, the raised bump having a radius smaller than the radius of the spherical head and a width greater than the width of the vertical slot.

2. The anchor of claim 1, wherein the vertical slot has an obround shape.

3. The anchor of claim 1, or 2, wherein the vertical slot is offset toward a top of the spherical head such that a top end of the vertical slot cuts deeper into the spherical head relative to a bottom end of the vertical slot.

4. The anchor of any one of the preceding claims, wherein the internal spherical pocket has a diameter greater than the width of the vertical slot, thereby forming an overhang beneath the sides of the vertical slot.

5. The anchor of any one of the preceding claims, wherein each side of the vertical slot defines symmetric lips.

6. The anchor of claim 5, wherein an apex of each lip is truncated with a flat.

7. The anchor of any one of the preceding claims, wherein the curved blade includes an elongate fin that ends away from a concave surface of the blade such that the elongate fin is generally orthogonal to the blade.

8. A spine stabilization system comprising:
an expandable implant having an upper endplate, a lower endplate, and an actuator assembly including a moveable anterior actuator, a moveable posterior actuator, and a stationary posterior actuator positioned between the upper and lower endplates to independently control anterior and posterior heights of the implant;
a bone anchor according to any one of the preceding claims that is insertable through the upper or lower endplate and into adjacent bone.t.

9. The spine stabilization system of claim 8, wherein the upper and/or lower endplates define a socket configured to receive the spherical head of the bone anchor, which guides the anchor into the adjacent bone.

10. The spine stabilization system of claim 9, wherein the socket has a partial spherical cut, which provides a congruent surface for the spherical head of the anchor to permit rotation of the anchor within the socket.

11. The spine stabilization system of claim 9 or 10, wherein the socket defines a stop, which engages with the raised bump to limit rotation of the anchor.

12. The spine stabilization system of claim 11, wherein the stop allows for up to 20 degrees of rotation for the anchor within the socket.

13. The spine stabilization system of any one of claims 8 - 12, wherein the expandable implant includes a blocking screw, which when rotated, covers a portion of the spherical head of the anchor, thereby preventing the anchor from backing out after insertion, and the raised bump prevents the non-threaded opening of the anchor from aligning with the blocking screw.
